# EUROPEAN PATENT APPLICATION

(11) **EP 4 759 344 A1**
(43) Date of publication of application: **17.06.2026**
(21) Application number: 24851201.4
(22) Date of filing: 02.08.2024
(51) Int. Cl.: A61M 25/09, A61M 25/01

(54) **MODULE FOR GUIDING AT LEAST ONE OF GUIDEWIRE AND CATHETER**

(30) Priority: 10.08.2023 KR 20230104896
(71) Applicant: XCATH, Inc., Houston, Texas 77054 (US)
(72) Inventor: FONSECA WARD, Eduardo Ramon Alejandro, Houston, Texas 77027 (US); JANG, Hyung Jun, Suwon-si, Gyeonggi-do 16420 (KR)
(74) Representative: Patentanwälte Ruff, Wilhelm, Beier, Dauster & Partner mbB
(86) International application number: PCT/IB2024/057490
(87) International publication number: WO 2025/032448

(57) **Abstract**

A module according to one embodiment of the present invention comprises: a housing 900 extending in a longitudinal direction from the front to the rear; a guide wire drive cassette 100 for pinching a guide wire 1 to drive the guide wire 1; at least one catheter drive cassette 200 for pinching a catheter 2 to drive the catheter 2; and, at least one catheter fixing cassette 300 to which a proximal end of the catheter 2 is coupled, wherein the module comprises a structure in which the guide wire drive cassette 100, the catheter drive cassette 200, and the catheter fixing cassette 300 are disposed in the housing 900, the guide wire drive cassette 100 comprises a guide wire drive module for rotating the guide wire 1 in a state where the guide wire 1 is pinched, the catheter drive cassette 200 comprises a catheter drive module for rotating the catheter 2 in a state where the catheter is pinched, and the guide wire drive cassette 100 and the catheter drive cassette 200 can be moved along the longitudinal direction of the housing 900 by their respective actuators.

## Description

### [TECHNICAL FIELD]

The present invention relates to a module for guiding at least one of a guide wire and a catheter used for minimally invasive interventional procedures.

### [BACKGROUND ART]

A guide wire is used to guide a secondary sheath (e.g., a catheter) along the guide wire to a desired location in the body, e.g., a mammalian body such as a human body. In one application, a guide wire is introduced into a body lumen, i.e., a blood vessel, through an incision through the patient's skin and lumen wall, and the introduced, or distal, end of the guide wire is guided therefrom to a desired location of the lumen, or in a desired location of a lumen which branches into, or from, the lumen into which the guide wire is introduced. Additionally, the catheter is guided along the guide wire.

One problem with guide wire and catheter introduction systems is that it is difficult to have the distal ends of the guide wire and catheter follow tortuous lumen geometry, or to guide the distal ends into an intersecting lumen or branch lumen connected to the lumen within which the distal ends of the guide wire and catheter is positioned. In some cases, a branch lumen the location of which is the intended destination of the distal ends of the guide wire and catheter intersects a lumen the distal end of which is at a large angle, for example, greater than 45 degrees, and in some cases, greater than 90 degrees.

To facilitate the orientation control of the distal ends of the guide wire and catheter, a robotic system is provided for reliably controlling movement of the guide wire and catheter. Also provided is a cassette to be used with the robotic system. Using the cassette, a guide wire and a catheter are guided to a desired location in the body by making forward, rearward, or rotational movements.

However, such a conventional cassette had problems that the guide wire and catheter could only perform any one of axial movement, such as forward movement and rearward movement, and rotational movement, and that axial movement, such as forward movement and rearward movement, and rotational movement could not be performed simultaneously.

### [PRIOR ART DOCUMENT]

### [PATENT DOCUMENT]

(PATENT DOCUMENT 1) US 2017-0274181 A1

### [DESCRIPTION OF THE INVENTION]

### [TECHNICAL PROBLEM]

A technical problem to be solved by the present invention is to provide a module for guiding at least one of a guide wire and a catheter, wherein the module allows at least one of the guide wire and catheter to perform axial movement, such as forward movement and rearward movement, and rotational movement, separately or simultaneously.

### [TECHNICAL MEANS FOR SOLVING THE PROBLEM]

A module according to one embodiment of the present invention comprises: a housing 900 extending in a longitudinal direction from the front to the rear; a guide wire drive cassette 100 for pinching a guide wire 1 to drive the guide wire 1; at least one catheter drive cassette 200 for pinching a catheter 2 to drive the catheter 2; and, at least one catheter fixing cassette 300 to which a proximal end of the catheter 2 is coupled, wherein the module comprises a structure in which the guide wire drive cassette 100, the catheter drive cassette 200, and the catheter fixing cassette 300 are disposed in the housing 900, the guide wire drive cassette 100 comprises a guide wire drive module for rotating the guide wire 1 in a state where the guide wire 1 is pinched, the catheter drive cassette 200 comprises a catheter drive module for rotating the catheter 2 in a state where the catheter is pinched, and the guide wire drive cassette 100 and the catheter drive cassette 200 can be moved along the longitudinal direction of the housing 900 by their respective actuators.

A module according to one embodiment of the present invention comprises a structure in which the guide wire drive module and the catheter drive module each comprise a rack 400, a pinion 500, a first bevel gear 600, a second bevel gear 700, and a pinch module 800.

A module according to one embodiment of the present invention comprises a structure in which the rack 400 is moved forward or rearward by rotation of the pinion 500, and when the rack 400 is moved rearward, the guide wire 1 or the catheter 2 is pinched by the pinch module 800 in conjunction with the movement of the rack 400, and when the rack 400 is moved forward, the guide wire 1 or the catheter 2 is released from being pinched by the pinch module 800 in conjunction with the movement of the rack 400.

A module according to one embodiment of the present invention comprises a structure in which, - in a state where the guide wire 1 is pinched by the pinch module 800 of the guide wire drive cassette 100, the guide wire 1 is rotated by rotation of the pinch module 800 of the guide wire drive cassette 100, and the guide wire 1 is moved forward or rearward by forward or rearward movement of the guide wire drive cassette 100 along the longitudinal direction of the housing 900, and - in a state where the catheter 2 is pinched by the pinch module 800 of the catheter drive cassette 200, the catheter 2 is rotated by rotation of the pinch module 800 of the catheter drive cassette 200, and the catheter 2 is moved forward or rearward by forward or rearward movement of the catheter drive cassette 200 along the longitudinal direction of the housing 900.

A module according to one embodiment of the present invention comprises a structure in which - only one of the forward or rearward movement of the guide wire 1 and the rotation of the guide wire 1 is performed, or both are performed simultaneously, and - only one of the forward or rearward movement of the catheter 2 and the rotation of the catheter 2 is performed, or both are performed simultaneously.

A module according to one embodiment of the present invention comprises a structure in which the guide wire drive cassette 100 is positioned at the rear of the catheter fixing cassette 300, and the catheter fixing cassette 300 is positioned at the rear of the catheter drive cassette 200.

A module according to one embodiment of the present invention comprises a structure in which, in a state where the catheter 2 is pinched by the pinch module 800 of the catheter drive cassette 200, the catheter fixing cassette 300 is moved forward or rearward in conjunction with forward or rearward movement of the catheter drive cassette 200.

A module according to one embodiment of the present invention comprises a structure in which, in a state where the catheter 2 is released from being pinched by the pinch module 800 of the catheter drive cassette 200, the catheter drive cassette 200 is moved forward or rearward independently of the catheter fixing cassette 300.

A module according to one embodiment of the present invention comprises a structure in which the diameter of the guide wire 1 is smaller than the diameter of the catheter 2, the guide wire 1 penetrates the interior of the catheter 2, and the driving of the guide wire 1 and the catheter 2 is performed independently of each other or in synchronization with each other.

A module according to one embodiment of the present invention comprises a structure in which the pinch module 800 is a collet structure 810 or a plate spring structure 820.

A module according to one embodiment of the present invention comprises a structure in which the collet structure 810 comprises a first portion 811, a second portion 812, and a collet module 813, the collet module 813 is disposed between the first portion 811 and the second portion 812, so as to face the inner side of the first portion 811 and be coupled to the second portion 812, when the rack 400 is moved rearward, the first portion 811 is moved rearward in conjunction with the movement of the rack 400, and when the first portion 811 is moved rearward, the inner side of the first portion 811 and the outer side of one or more collets 814 of the collet module 813 come into contact with each other at one or more contact points, so that the collet 814 is moved in a direction toward the center 810A of the structure 810, and the guide wire 1 or the catheter 2 penetrating the center 810A of the collet structure 810 is pinched by the collet 814.

A module according to one embodiment of the present invention comprises a structure in which the inner side of the first portion 811 and the outer side of one or more collets 814 of the collet module 813 come into contact with each other at at least two contact points 814A, 814B, and the at least two contact points 814A, 814B are spaced apart from each other along the longitudinal direction of the collet 814 on the outer side of the collet 814.

A module according to one embodiment of the present invention comprises a structure in which at least one anti-slip groove 814C is formed along the longitudinal direction of the collet 814 on the inner side of the collet 814.

A module according to one embodiment of the present invention comprises a structure in which the plate spring structure 820 comprises a moving portion 821, a supporting portion 822, and one or more plate springs 823, the plate spring 823 is disposed between the moving portion 821 and the supporting portion 822, such that a first end 823A of the plate spring 823 is fixed to an inner surface of the moving portion 821, and a second end 823B of the plate spring 823 is fixed to an inner surface of the supporting portion 822, when the rack 400 is moved rearward, the moving portion 821 is moved rearward in conjunction with the movement of the rack 400, and when the moving portion 821 is moved rearward, the plate springs 823 are subjected to bending deformation in a direction toward each other, so that the guide wire 1 or the catheter 2 penetrating the center 810A of the plate spring structure 820 is pinched.

A module according to one embodiment of the present invention comprises a structure in which the degree of the bending deformation of the plate spring 823 increases as the distance by which the rack 400 is moved rearward increases.

In a module according to one embodiment of the present invention, a guide wire supply line 101 through which the guide wire 1 may be supplied is formed in the guide wire drive cassette 100, and the guide wire supply line 101 comprises an inlet portion 101A where the guide wire 1 enters, an outlet portion 101B where the guide wire 1 exits, a curved portion 101C between the inlet portion 101A and the outlet portion 101B, and a protruding portion 101D.

A module according to one embodiment of the present invention comprises a structure in which the distance by which the guide wire drive cassette 100 and the catheter drive cassette 200 can move forward or rearward along the longitudinal direction of the housing 900 is limited to a distance at which the guide wire 1 or the catheter 2 does not buckle.

A module according to one embodiment of the present invention comprises a structure in which the at least one catheter drive cassette 200 comprises a plurality of catheter drive cassettes, and the at least one catheter fixing cassette 300 comprises a plurality of catheter fixing cassettes.

A module according to one embodiment of the present invention comprises a structure in which the at least one catheter drive cassette 200 comprises one first catheter drive cassette 210 and one second catheter drive cassette 220, the at least one catheter fixing cassette 300 comprises one first catheter fixing cassette 310 and one second catheter fixing cassette 320, the guide wire drive cassette 100 is disposed at the rear of the first catheter fixing cassette 310, the first catheter fixing cassette 310 is disposed at the rear of the first catheter drive cassette 210, the first catheter drive cassette 210 is disposed at the rear of the second catheter fixing cassette 320, the second catheter fixing cassette 320 is disposed at the rear of the second catheter drive cassette 220, a proximal end of the first catheter is coupled to the first catheter fixing cassette 310, a proximal end of the second catheter is coupled to the second catheter fixing cassette 320, the diameter of the guide wire 1 is smaller than the diameter of the first catheter, the guide wire 1 penetrates the interior of the first catheter, the diameter of the first catheter is smaller than the diameter of the second catheter, and the first catheter penetrates the interior of the second catheter.

A module according to one embodiment of the present invention comprises a structure in which the at least one catheter fixing cassette 300 comprises one third catheter fixing cassette 330, the third catheter fixing cassette 330 is disposed at a front end of the housing 900, the second catheter drive cassette 220 is disposed at the rear of the third catheter fixing cassette 330, a proximal end of the third catheter is coupled to the third catheter fixing cassette, the diameter of the second catheter is smaller than the diameter of the third catheter, and the second catheter penetrates the interior of the third catheter.

### [EFFECT OF THE INVENTION]

According to the module of the present invention, at least one of the guide wire and the catheter guided by the module can perform axial movement, such as forward movement and rearward movement, and rotational movement, separately or simultaneously, so that the position of the distal ends of the guide wire and the catheter can be easily and accurately controlled, thereby effectively reducing the time taken for procedures including minimally invasive intervention procedures.

### [BRIEF DESCRIPTION OF THE DRAWINGS]

FIG. 1 is a plan view of a module for guiding at least one of a guide wire and a catheter according to one embodiment of the present invention, illustrating paths of the guide wire and the catheter.
FIG. 2A is a cross-sectional view of a catheter drive module or a guide wire drive module in a state of release from pinching in a module according to one embodiment of the present invention.
FIG. 2B is a plan view of a catheter drive module or a guide wire drive module in a state of release from pinching in a module according to one embodiment of the present invention.
FIG. 3A is a cross-sectional view of a catheter drive module or a guide wire drive module in a state of pinching in a module according to one embodiment of the present invention.
FIG. 3B is a plan view of a catheter drive module or a guide wire drive module in a state pinching in a module according to one embodiment of the present invention.
FIG. 4 is a plan view of a catheter drive module or a guide wire drive module in a state of making rotational movement in a module according to one embodiment of the present invention.
FIG. 5A is a plan view of a catheter drive cassette or a guide wire drive cassette before forward movement in a module according to one embodiment of the present invention.
FIG. 5B is a plan view of a catheter drive cassette or a guide wire drive cassette after forward movement in a module according to one embodiment of the present invention.
FIG. 6 is a perspective view of a collet structure according to one embodiment of the present invention.
FIG. 7A is a top view of a collet structure in a state of release from pinching in a module according to one embodiment of the present invention.
FIG. 7B is a side view of a collet structure in a state of release from pinching in a module according to one embodiment of the present invention.
FIG. 8 is a cross-sectional view of a collet structure in a state of release from pinching in a module according to one embodiment of the present invention.
FIG. 9A is a top view of a collet structure in a state of pinching in a module according to one embodiment of the present invention.
FIG. 9B is a side view of a collet structure in a state of pinching in a module according to one embodiment of the present invention.
FIG. 10 is a cross-sectional view of a collet structure in a state of pinching in a module according to one embodiment of the present invention.
FIG. 11 is an enlarged view of an anti-slip groove of a collet in a module according to one embodiment of the present invention.
FIG. 12 is a cross-sectional view of a collet structure in a state of release from pinching in a module according to a modified embodiment of the present invention.
FIG. 13 is a perspective view of a plate spring structure in a module according to one embodiment of the present invention.
FIG. 14 is a perspective view of a plate spring structure before being assembled in a module according to one embodiment of the present invention.
FIG. 15 is a cross-sectional view of a spring structure in a state of release from pinching in a module according to a modified embodiment of the present invention
FIG. 16 is a cross-sectional view of a plate spring structure in a state of pinching in a module according to one embodiment of the present invention.
FIG. 17 is a perspective view of a guide wire drive cassette in a module according to one embodiment of the present invention.

### [BEST EMBODIMENTS TO CARRY OUT THE INVENTION]

Hereinafter, preferred examples of the present invention will be described with reference to the attached drawings.

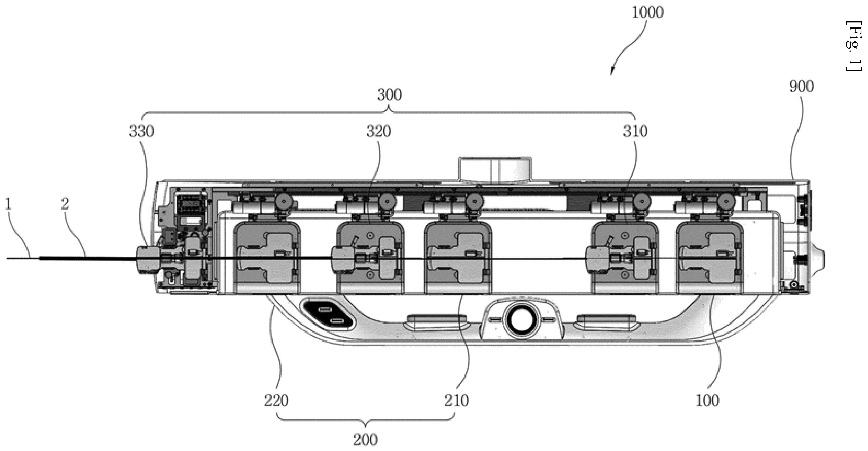

FIG. 1 is a plan view of a module for guiding at least one of a guide wire and a catheter according to one embodiment of the present invention, illustrating paths of the guide wire and the catheter.

In FIG. 1, the side on which the guide wire 1 and the catheter 2 protrude from the interior of the housing 900 is referred to as the front, and the side opposite to the side on which the guide wire 1 and the catheter 2 protrude from the interior of the housing 900 is referred to as the rear.

Referring to FIG. 1, a module 1000 for guiding at least one of a guide wire 1 and a catheter 2 comprises a housing 900 extending in a longitudinal direction from the front to the rear; a guide wire drive cassette 100 for pinching the guide wire 1 to drive the guide wire 1, at least one catheter drive cassette 200 for pinching the catheter 2 to drive the catheter 2, and at least one catheter fixing cassette 300 to which a proximal end of the catheter 2 is coupled.

The guide wire drive cassette 100 and the catheter drive cassette 200 are configured to be moved forward or rearward in the housing 900 by predetermined actuators.

Among the catheter fixing cassettes 300, the first catheter fixing cassette 310 and the second catheter fixing cassette 320 are configured to be moved forward or rearward in the housing 900 by predetermined actuators.

However, among the catheter fixing cassettes 300, the third catheter fixing cassette 330 is configured not to be moved in the housing 900 and is fixed to the front end of the housing 900.

The proximal end of the catheter 2 coupled to the catheter fixing cassette 300 remains fixed. However, the fixed proximal end of the catheter 2 may be released by the operator's manipulation.

The guide wire 1, which is a flexible wire-shaped feature, is introduced into a blood vessel through an incision in the patient's skin, and makes forward, rearward, or rotational movement within the blood vessel by manipulation of the module 1000 by the operator.

As in the case of the guide wire 1, the catheter 2, which is a flexible tube-shaped feature, is introduced into a blood vessel through an incision in the patient's skin, and makes forward, rearward, or rotational movement within the blood vessel by manipulation of the module 1000 by the operator.

The catheter 2 has a tube shape having an outer diameter and an inner diameter, and the diameter of the guide wire 1 is smaller than the inner diameter of the catheter 2 so that the guide wire 1 can penetrate the inner diameter of the catheter 2.

The guide wire drive cassette 100 pinching the guide wire 1 means that the guide wire 1 is fixed by a predetermined pinch module included in the guide wire drive cassette 100.

In a state where the guide wire 1 is pinched, the guide wire 1 is driven (makes forward, rearward, or rotational movement) as the guide wire drive cassette 100 is driven (makes forward, rearward, or rotational movement).

The guide wire drive cassette 100 releasing the guide wire 1 from pinching means that the guide wire 1 transitions from a state of being fixed by a predetermined pinch module included in the guide wire drive cassette 100 to a state of being released from the fixing.

In a state where the guide wire 1 is released from pinching, even if the guide wire drive cassette 100 is driven (makes forward, rearward, or rotational movement), the guide wire 1 is not driven (does not make forward, rearward, or rotational movement). However, in a state where the guide wire 1 is released from pinching, the guide wire 1 may be driven (make forward, rearward, or rotational movement) by manual manipulation by the operator.

The catheter drive cassette 200 pinching the catheter 2 means that the catheter 2 is fixed by a predetermined pinch module included in the catheter drive cassette 200.

In a state where the catheter 2 is pinched, the catheter 2 is driven (makes forward, rearward, or rotational movement) as the catheter drive cassette 200 is driven (makes forward, rearward, or rotational movement).

The catheter drive cassette 200 releasing the catheter 2 from pinching means that the catheter 2 transitions from a state of being fixed by a predetermined pinch module included in the catheter drive cassette 200 to a state of being released from the fixing.

In a state where the catheter 2 is released from pinching, even if the catheter drive cassette 200 is driven (makes forward, rearward, or rotational movement), the catheter 2 is not driven (does not make forward, rearward, or rotational movement). However, in a state where the catheter 2 is released from pinching, the catheter 2 may be driven (make forward, rearward, or rotational movement) by manual manipulation by the operator.

The guide wire drive cassette 100, the catheter drive cassette 200, and the catheter fixing cassette 300 are disposed in the housing 900.

The guide wire drive cassette 100 comprises a guide wire drive module for rotating the guide wire 1 in a state where the guide wire 1 is pinched, and the catheter drive cassette 200 comprises a catheter drive module for rotating the catheter 2 in a state where the catheter 2 is pinched.

The guide wire drive cassette 100 and the catheter drive cassette 200 are moved forward or rearward along the longitudinal direction of the housing 900 by their respective actuators (not shown).

The guide wire drive cassette 100 and the catheter drive cassette 200 make rotational movement about the longitudinal direction of the housing 900 as an axis by their respective actuators (not shown).

The driving (forward, rearward, or rotational movement) of the guide wire drive cassette 100 and the catheter drive cassette 200 may be performed independently of each other or in synchronization with each other.

The guide wire 1 makes forward, rearward, or rotational movement by the driving (forward, rearward, or rotational movement) of the guide wire drive cassette 100.

The catheter 2 makes forward, rearward, or rotational movement by the driving (forward, rearward, or rotational movement) of the catheter drive cassette 200.

The driving of the guide wire 1 and the catheter 2 may be performed independently of each other or in synchronization with each other.

Regarding the housing 900 extending in a longitudinal direction from the front to the rear, the guide wire 1 and the catheter 2 are penetrated from the front end of the housing 900 along the longitudinal direction.

The interior of the guide wire drive cassette 100 is penetrated by the guide wire 1, and the interior of the catheter drive cassette 200 is penetrated by the catheter 2.

Furthermore, the guide wire 1 and the catheter 2 are configured as flexible tube-shaped members, the diameter of the guide wire 1 is smaller than the diameter of the catheter 2, and the interior of the catheter 2 is penetrated by the guide wire 1.

The at least one catheter drive cassette 200 comprises a plurality of catheter drive cassettes, and the at least one catheter fixing cassette 300 comprises a plurality of catheter fixing cassettes.

In an example of a module according to one embodiment of the present invention, the at least one catheter drive cassette 200 comprises one first catheter drive cassette 210 and one second catheter drive cassette 220, and at least one catheter fixing cassette 300 comprises one first catheter fixing cassette 310 and one second catheter fixing cassette 320.

The guide wire drive cassette 100 is disposed at the rear of the first catheter fixing cassette 310, the first catheter fixing cassette 310 is disposed at the rear of the first catheter drive cassette 210, the first catheter drive cassette 210 is disposed at the rear of the second catheter fixing cassette 320, and the second catheter fixing cassette 320 is disposed at the rear of the second catheter drive cassette 220.

The first catheter fixing cassette 310 is configured to have a proximal end of the first catheter attached and fixed thereto, and the second catheter fixing cassette 320 is configured to have a proximal end of the second catheter attached and fixed thereto.

Attaching and fixing the proximal ends of the first catheter and the second catheter to the first catheter fixing cassette 310 and the second catheter fixing cassette 320 is performed by, for example, a threaded coupling means.

Therefore, the guide wire 1 can be inserted into the proximal end of the first catheter coupled to the first catheter fixing cassette 310, and the first catheter can be inserted into the proximal end of the second catheter coupled to the second catheter fixing cassette 320. Here, the proximal end of the guide wire 1 or the catheter 2 means a portion of the guide wire 1 or the catheter 2 that is not positioned in or inserted into the body.

Since the diameter of the guide wire 1 is smaller than the inner diameter of the first catheter, the guide wire 1 may penetrate the inner diameter of the first catheter at the proximal end of the first catheter and extend beyond the distal end of the first catheter, which is on the opposite side thereof.

In addition, since the outer diameter of the first catheter is smaller than the inner diameter of the second catheter, the first catheter may penetrate the inner diameter of the second catheter at the proximal end of the second catheter and extend beyond the distal end of the second catheter, which is on the opposite side thereof.

In addition, in an example of a module according to one embodiment of the present invention, at least one catheter fixing cassette 300 further comprises one third catheter fixing cassette 330.

The third catheter fixing cassette 330 is configured to have a proximal end of the third catheter attached and fixed thereto. Here, attaching and fixing the proximal end of the third catheter to the third catheter fixing cassette 330 is performed by, for example, a threaded coupling means.

The second catheter drive cassette 220 is disposed at the rear of the third catheter fixing cassette 330, i.e., adjacent to the proximal end of the third catheter.

Since the outer diameter of the second catheter is smaller than the inner diameter of the third catheter, the second catheter may penetrate the inner diameter of the third catheter at the proximal end of the third catheter and extend beyond the distal end of the third catheter, which is on the opposite side thereof.

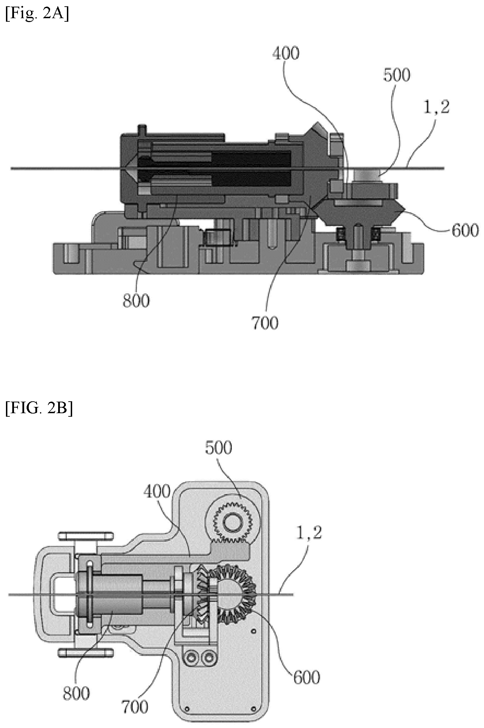

FIG. 2A is a cross-sectional view of a catheter drive module or a guide wire drive module in a state of release from pinching in a module according to one embodiment of the present invention.

FIG. 2B is a plan view of a catheter drive module or a guide wire drive module in a state of release from pinching in a module according to one embodiment of the present invention.

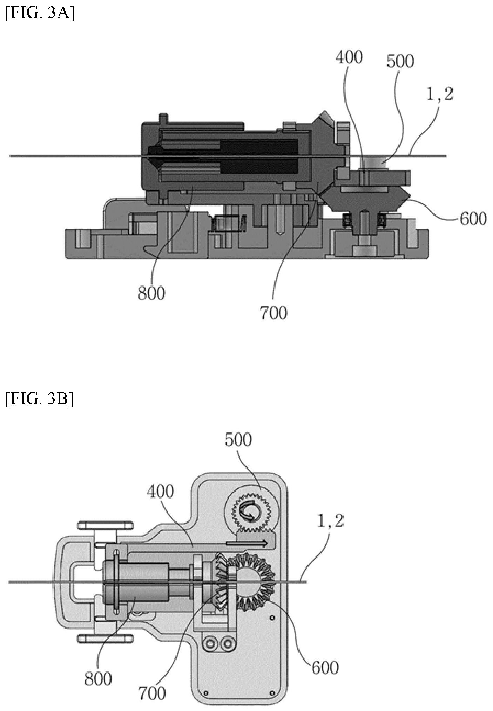

FIG. 3A is a cross-sectional view of a catheter drive module or a guide wire drive module in a state of pinching in a module according to one embodiment of the present invention.

FIG. 3B is a plan view of a catheter drive module or a guide wire drive module in a state pinching in a module according to one embodiment of the present invention.

Referring to FIGS. 2A, 2B, 3A, and 3B, the guide wire drive module included in the guide wire drive cassette 100 and the catheter drive module included in the catheter drive cassette 200 each comprise a rack 400, a pinion 500, a first bevel gear 600, a second bevel gear 700, and a pinch module 800.

Regarding the guide wire drive module and the catheter drive module, the side on which the pinch module 800 is positioned is referred to as the front, and the side on which first bevel gear 600 is positioned is referred to as the rear.

However, the structures of the guide wire drive module included in the guide wire drive cassette 100 and the catheter drive module included in the catheter drive cassette 200 may differ from each other.

A series of splines, typically disposed along a straight path of the rack 400, sequentially engage with gear teeth of the pinion 500 to cause linear movement of the rack 400 in conjunction with rotation of the pinion 500 about the drive shaft of the pinion 500, as known to those skilled in the art. The rack 400 extends in the longitudinal direction from the front side toward the rear side of the guide wire drive module or the catheter drive module.

The pinion 500 is rotated about a pinion shaft, which is rotated by a separate feature (not shown). The pinion 500 is rotated in conjunction with the rotation of the pinion shaft.

The rack 400 is moved forward or rearward by the rotation of the pinion 500.

When the rack 400 is moved rearward, a portion of the pinch module 800 is moved rearward in conjunction with the rearward movement of the rack 400, which reduces the width of the pinch through which the guide wire 1 or the catheter 2 penetrates to cause the guide wire 1 or the catheter 2 to be pinched or held by the pinch module 800.

The pinch module 800 is any one of a collet structure 810 or a plate spring structure 820.

When the rack 400 is moved forward, a portion of the pinch module 800 is moved forward in conjunction with the forward movement of the rack 400, which increases the width of the pinch through which the guide wire 1 or the catheter 2 penetrates to release the guide wire 1 or the catheter 2 from being pinched or held by the pinch module 800.

The first bevel gear 600 and the second bevel gear 700 engage with each other. A rotation shaft of the first bevel gear 600 and a rotation shaft of the second bevel gear 700 may be disposed, for example, at an angle of 90 degrees with respect to each other.

Ends of the second bevel gear 700 and the pinch module 800 adjacent to the second bevel gear 700 are mechanically coupled to each other or formed as an integral feature.

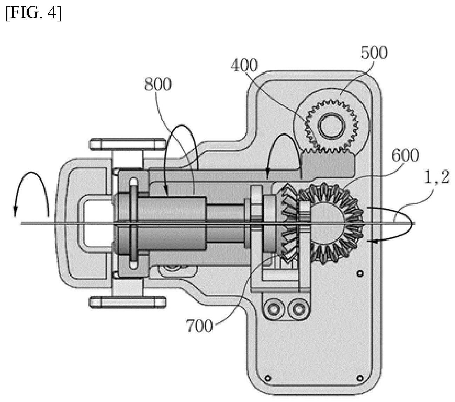

FIG. 4 is a plan view of a catheter drive module or a guide wire drive module in a state of making rotational movement in a module according to one embodiment of the present invention.

Referring to FIG. 4, the first bevel gear 600 is rotated about the shaft of the first bevel gear 600, and the shaft of the first bevel gear 600 is rotated by a separate feature (not shown). The second bevel gear 700 is rotated according to the rotation of the first bevel gear 600, and the pinch module 800 is rotated according to the rotation of the second bevel gear 700.

In addition, the rotational movement of the guide wire 1 and the catheter 2 is performed on the premise that the guide wire 1 and the catheter 2 are pinched or held by the pinch module 800.

That is, the guide wire 1, in a state being pinched or held by the pinch module 800 of the guide wire drive module included in the guide wire drive cassette 100, is rotated according to the rotation of the pinch module 800.

In addition, the catheter 2, in a state of being pinched or held by the pinch module 800 of the catheter drive module included in the catheter drive cassette 200, is rotated according to the rotation of the pinch module 800.

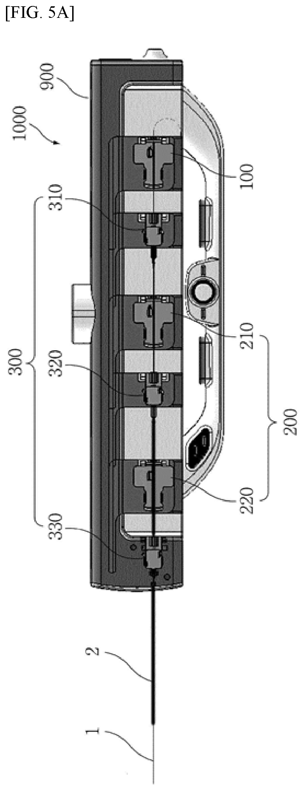

FIG. 5A is a plan view of a catheter drive cassette or a guide wire drive cassette before forward movement in a module according to one embodiment of the present invention.

In FIG. 5A, the side on which the guide wire 1 and the catheter 2 protrude from the interior of the housing 900 is referred to as the front, and the side opposite to the side on which the guide wire 1 and the catheter 2 protrude from the interior of the housing 900 is referred to as the rear.

Referring to FIG. 5A, the guide wire drive cassette 100 is disposed at the rear of the catheter fixing cassette 300, i.e., the proximal end or side thereof, while being spaced apart from the catheter fixing cassette 300 by a predetermined distance. The catheter fixing cassette 300 is disposed at the rear of the catheter drive cassette 200, i.e., the proximal end or side thereof, while being spaced apart from the catheter drive cassette 200 by a predetermined distance.

Specifically, the guide wire drive cassette 100 is disposed at the rear of the first catheter fixing cassette 310 while being spaced apart from the first catheter fixing cassette 310 by a predetermined distance. Since the diameter of the guide wire 1 is smaller than the inner diameter of the first catheter, the guide wire 1 may penetrate the interior of the first catheter and move forward or rearward.

Since the guide wire 1, which is a flexible wire-shaped feature, is not highly rigid, there is a possibility that, when it is moved forward or rearward in a state where the proximal end of the guide wire 1 is pinched or held, buckling occurs if the distance from the distal end of the guide wire 1 to the proximal end of the guide wire 1 is a predetermined distance or more. Therefore, the predetermined distance by which the guide wire drive cassette 100 is spaced from the first catheter fixing cassette 310 is limited to a distance at which the guide wire 1 does not buckle when the guide wire 1 moves from the proximal end direction to the distal end direction, i.e., when inserted into the body, or when penetrating the interior of the catheter 1. For example, the distance at which the guide wire 1 does not buckle is 40 mm to 60 mm, and preferably 50 mm. However, the distance at which the guide wire 1 does not buckle varies depending on the rigidity of the material constituting the guide wire 1.

The first catheter drive cassette 210 is disposed at the rear of the second catheter fixing cassette 320 while being spaced apart from the second catheter fixing cassette 320 by a predetermined distance. Since the outer diameter of the first catheter is smaller than the inner diameter of the second catheter, the first catheter can move forward or rearward by penetrating the interior of the second catheter.

Since the first catheter, which is a flexible tube-shaped feature, is not highly rigid, there is a possibility that, when it is moved forward or rearward in a state where the proximal end of the first catheter is pinched or held, buckling occurs if the distance from the distal end of the first catheter to the proximal end of the first catheter is a predetermined distance or more. Therefore, the predetermined distance by which the first catheter drive cassette 210 is spaced from the second catheter fixing cassette 320 is limited to a distance at which the first catheter does not buckle when the first catheter moves by penetrating the interior of the second catheter. For example, the distance at which the first catheter does not buckle is 40 mm to 60 mm, and preferably 50 mm. However, the distance at which the first catheter does not buckle varies depending on the rigidity of the material constituting the first catheter.

The second catheter drive cassette 220 is disposed at the rear of the third catheter fixing cassette 330 while being spaced from the third catheter fixing cassette 330 by a predetermined distance. Since the outer diameter of the second catheter is smaller than the inner diameter of the third catheter, the second catheter can move forward or rearward by penetrating the interior of the third catheter.

Since the second catheter, which a flexible tube-shaped feature, is not highly rigid, there is a possibility that, when it is moved forward or rearward in a state where the proximal end of the second catheter is pinched or held, buckling occurs if the distance from the distal end of the second catheter to the proximal end of the second catheter is a predetermined distance or more. Therefore, the predetermined distance by which the second catheter drive cassette 220 is spaced from the third catheter fixing cassette 330 is limited to a distance at which the second catheter does not buckle when the second catheter moves by penetrating the interior of the third catheter. For example, the distance at which the second catheter does not buckle is 40 mm to 60 mm, and preferably 50 mm. However, the distance at which the second catheter does not buckle varies depending on the rigidity of the material constituting the second catheter.

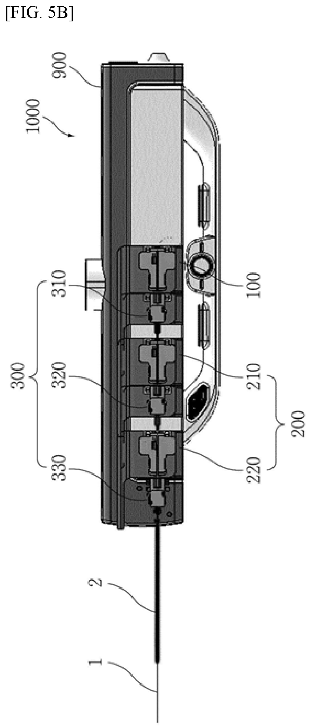

FIG. 5B is a plan view of a catheter drive cassette or a guide wire drive cassette after forward movement in a module according to one embodiment of the present invention.

In FIG. 5B, the side on which the guide wire 1 and the catheter 2 protrude from the interior of the housing 900 is referred to as the front, and the side opposite to the side on which the guide wire 1 and the catheter 2 protrude from the interior of the housing 900 is referred to as the rear.

Referring to FIG. 5B, the guide wire drive cassette 100 is moved forward or rearward by an actuator (not shown) by a distance that is equal to or less than the predetermined distance by which it is spaced apart from the catheter fixing cassette 300, and the catheter drive cassette 200 is moved forward or rearward by a distance that is equal to or less than the predetermined distance by which it is spaced apart from the catheter fixing cassette 300.

In a state in which the catheter 2 is pinched by the catheter drive cassette 200, the catheter 2 is moved forward or rearward in conjunction with the forward or rearward movement of the catheter drive cassette 200.

In a state in which the catheter 2 is released from being pinched by the catheter drive cassette 200, the catheter drive cassette 200 is moved forward or rearward independently of the catheter 2.

Specifically, the guide wire drive cassette 100 is moved forward or rearward by an actuator (not shown) by a distance that is equal to or less than the predetermined distance by which it is spaced apart from the catheter fixing cassette 300. Here, when the guide wire drive cassette 100 is moved in a state where it has pinched the guide wire 1, the guide wire 1 is moved forward or rearward while maintaining a non-buckled state.

The first catheter drive cassette 210 is moved forward or rearward by an actuator (not shown) by a distance that is equal to or less than the predetermined distance by which it is spaced apart from the second catheter fixing cassette 320. Here, when the first catheter drive cassette 210 is moved in a state where it has pinched the first catheter, the first catheter is moved forward or rearward while maintaining a non-buckled state. In addition, when the first catheter drive cassette 210 is moved in a state where it has pinched the first catheter, the first catheter fixing cassette 310 is moved forward or rearward in conjunction with the movement of the first catheter drive cassette 210. However, when the first catheter drive cassette 210 is moved in a state in which it has released the first catheter from pinching, the first catheter drive cassette 210 is moved forward or rearward independently of the first catheter fixing cassette 310.

The second catheter drive cassette 220 is moved forward or rearward by an actuator (not shown) by a distance that is equal to or less than the predetermined distance by which it is spaced apart from the third catheter fixing cassette 330. Here, when the second catheter drive cassette 220 is moved in a state where it has pinched the second catheter, the second catheter is moved forward or rearward while maintaining a non-buckled state. In addition, when the second catheter drive cassette 220 is moved in a state where it has pinched the second catheter, the second catheter fixing cassette 320 is moved forward or rearward in conjunction with the movement of the second catheter drive cassette 220. However, when the second catheter drive cassette 220 is moved in a state in which it has released the second catheter from pinching, the second catheter drive cassette 220 is moved forward or rearward independently of the second catheter fixing cassette 320.

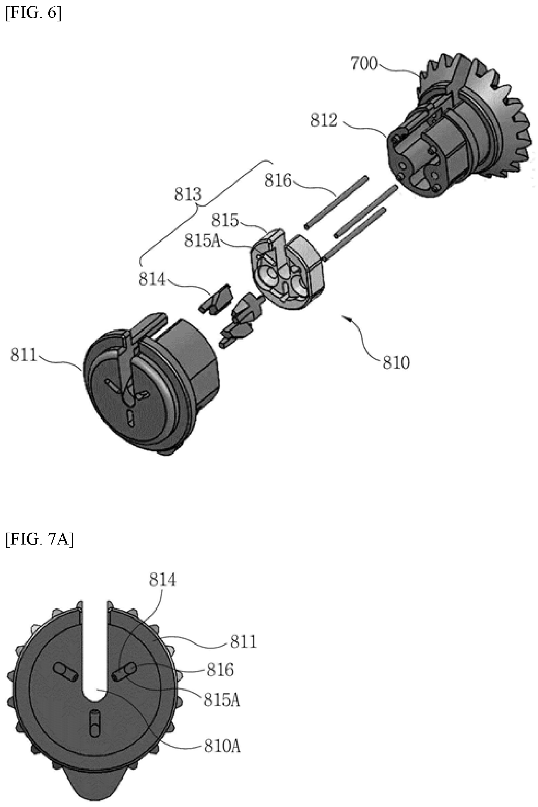

FIG. 6 is a perspective view of a collet structure according to one embodiment of the present invention.

FIG. 7A is a top view of a collet structure in a state of release from pinching in a module according to one embodiment of the present invention.

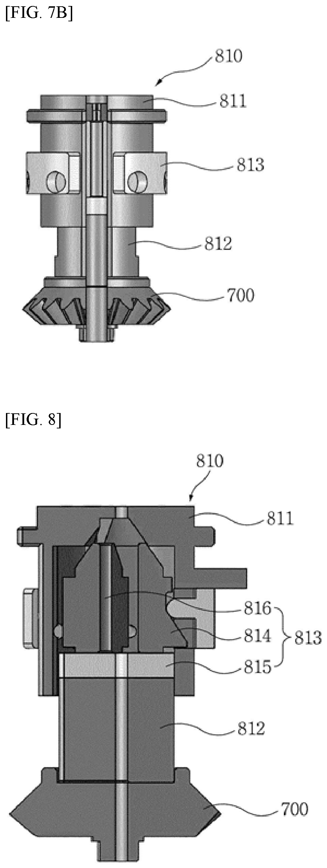

FIG. 7B is a side view of a collet structure in a state of release from pinching in a module according to one embodiment of the present invention.

FIG. 8 is a cross-sectional view of a collet structure in a state of release from pinching in a module according to one embodiment of the present invention.

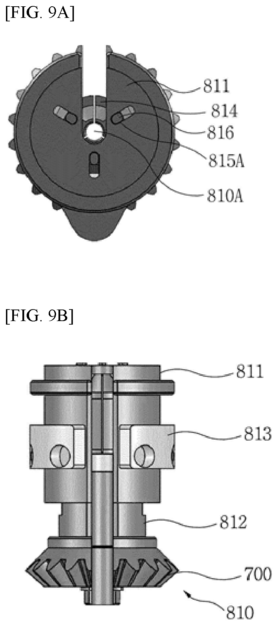

FIG. 9A is a top view of a collet structure in a state of pinching in a module according to one embodiment of the present invention.

FIG. 9B is a side view of a collet structure in a state of pinching in a module according to one embodiment of the present invention.

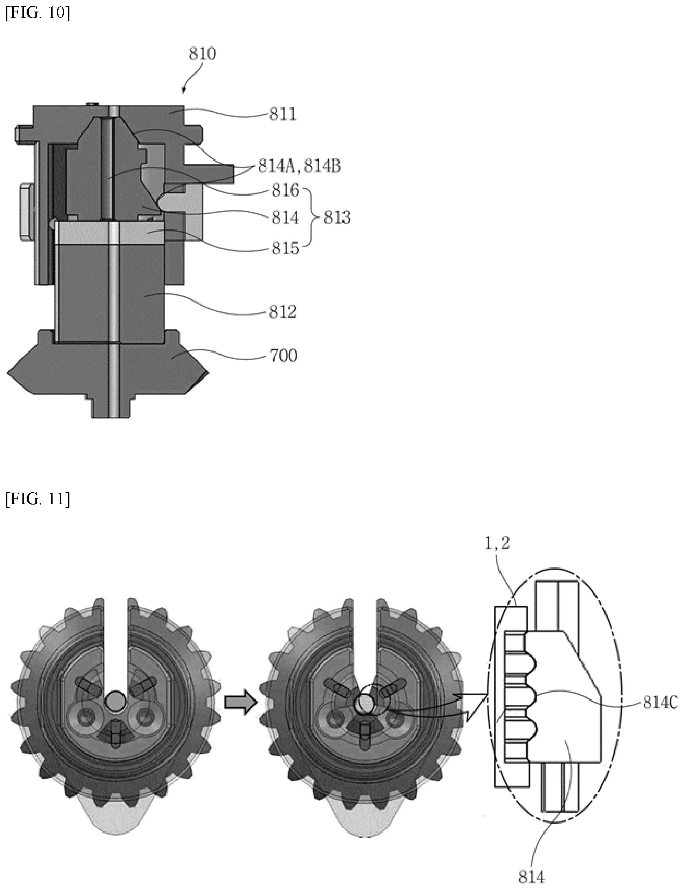

FIG. 10 is a cross-sectional view of a collet structure in a state of pinching in a module according to one embodiment of the present invention.

FIG. 11 is an enlarged view of an anti-slip groove of a collet in a module according to one embodiment of the present invention.

Referring to FIGS. 6 to 11, the collet structure 810 comprises a first portion 811, a second portion 812, and a collet module 813.

The collet module 813 is disposed between the first portion 811 and the second portion 812, so as to face the inner side of the first portion 811, and be coupled with the second portion 812.

In the collet structure 810, the side on which the first portion 811 is positioned is referred to as the front, and the side on which the second portion 812 is positioned is referred to as the rear.

Since the first portion 811 is coupled to a straight extended portion of the rack 400, when the rack 400 is moved rearward, the first portion 811 is also moved rearward, and when the rack 400 is moved forward, the first portion 811 is also moved forward.

The second portion 812 is mechanically coupled to the second bevel gear 700, or the second portion 812 is formed as a feature integral with the second bevel gear 700. Due to the structure, the second portion 812 is rotated together with rotation of the second bevel gear 700. The collet module 813 is rotated together with rotation of the second portion 812.

The collet module 813 comprises at least one collet 814, a guide plate 815, and at least one biasing member 816.

The at least one collet 814 is two or more collets 814, three or more collets 814, and preferably three collets 814.

The at least one biasing member 816 is formed of an elastic material and has a restoring force that resists an external force. For example, the biasing member 816 is an elastic spring.

A guide groove 815A is formed along a radius in a slot shape from the center toward the outer circumferential direction of the guide plate 815, a portion of the collet 814 is disposed in the guide groove 815A, the biasing member 816 is disposed on the outer circumferential side of the guide groove 815A by penetrating the portion of the collet 814, and when the collet 814 moves from the outer circumference to the center of the guide plate 815 along the guide groove 815A, the biasing member 816 imparts, to the collet 814, a restoring force in a direction from the center toward the outer circumferential direction of the guide plate 815. Here, the center of the guide plate 815 coincides with the center 810A of the collet structure 810.

The first portion 811, the second portion 812, and the guide plate 815 are coupled by the biasing member 816 penetrating the guide groove 815A of the guide plate 815. When the rack 400 is moved rearward, the first portion 811 is moved rearward in conjunction with the rearward movement of the rack 400.

Before the rack 400 is moved rearward, the gap between the first portion 811 and the second portion 812 is large as shown in FIGS. 7B and 8, such that the tapered side surface on the inner side of the first portion 811 and the tapered side surface on the outer side of the collet 814 do not come into contact with each other.

At this time, since the collets 814 are not moved as shown in FIG. 7A, the gaps between the collets 814 and the center 810A of the collet structure 810 are not reduced, thereby not pinching the guide wire 1 or the catheter 2 penetrating the center 810A of the collet structure 810.

When the first portion 811 is moved rearward, the gap between the first portion 811 and the second portion 812 is reduced as shown in FIGS. 9B and 10 and the tapered side surface on the inner side of the first portion 811 and the tapered side surface on the outer side of the collet 814 come into contact with each other at one or more contact points, the tapered side surface on the outer side of the collet 814 slides along the tapered side surface on the inner side of the first portion 811, and the collet 814 is moved in a direction toward the center 810A of the collet structure 810.

At this time, since the collet 814 is moved along the slot-shaped guide groove 815A formed from the outer circumference to the center of the guide plate 815 as shown in FIG. 9A, the gaps between the collets 814 and the center 810A of the collet structure 810 are reduced, thereby pinching the guide wire 1 or the catheter 2 penetrating the center 810A of the collet structure 810.

The tapered side surface on the inner side of the first portion 811 and the tapered side surface on the outer side of the collet 814 come into contact with each other at at least two contact points 814A, 814B.

The at least two contact points 814A, 814B are spaced apart from each other along the longitudinal direction of the collet 814 on the tapered side surface on the outer side of the collet 814.

Referring to FIG. 11, at least one anti-slip groove 814C is formed along the longitudinal direction of the collet 814 on the inner side of the collet 814 where the guide wire 1 or the catheter 2 is pinched. There are flat surfaces between the anti-slip grooves 814C, and the groove depths of the anti-slip grooves 814C are the same. Due to the anti-slip grooves 814C, the collet 814 has the effect of increasing the contact area with the pinched guide wire 1 or catheter 2.

Specifically, since the guide wire 1 or the catheter 2 are flexible features and can be deformed due to elasticity, when the collet 814 pinches the guide wire 1 or the catheter 2, a flat surface between anti-slip grooves 814C comes into contact with the guide wire 1 or the catheter 2 first, and then, due to the shape deformation of the guide wire 1 or the catheter 2, the guide wire 1 or the catheter 2 is inserted into and comes into contact with an anti-slip groove 814C.

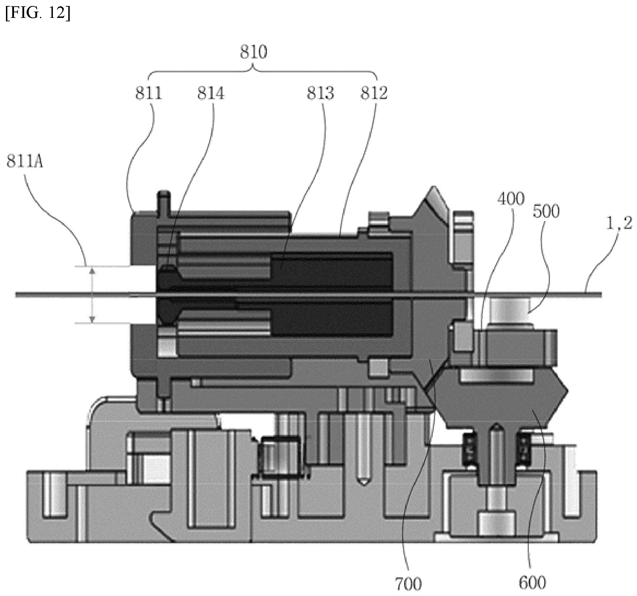

Therefore, the collet 814 has the effect of increasing the contact area with the guide wire 1 or the catheter 2. The effect is more evident when pinching the catheter 2, which has a shape of a flexible tube with a relatively large diameter and easily subjected to shape deformation. FIG. 12 is a cross-sectional view of a collet structure in a state of release from pinching in a module according to a modified embodiment of the present invention.

The collet structure 810 illustrated in FIG. 12 differs from the collet structure 810 illustrated in FIGS. 6 to 11 in that a through hole 811A having a diameter smaller than the outer diameter of the collet 814 is formed in the first portion 811.

Referring to FIG. 12, in the collet structure 810, the side on which the first portion 811 is positioned is referred to as the front, and the side on which the second portion 812 is positioned is referred to as the rear.

A through hole 811A having a diameter smaller than the outer diameter of the collet 814 is formed at the front end of the first portion 811 of the collet structure 810.

When the rack 400 is moved rearward, the first portion 811 is moved rearward in conjunction with the rearward movement of the rack 400, and when the first portion 811 is moved rearward, i.e., when the first portion 811 and the second portion 812 come closer to each other, the outer portion of the collet 814 with a large diameter is fitted into the through hole 811A of the first portion 811.

Accordingly, the inner side of the through hole 811A of the first portion 811 and the outer portion of the collet 814 come into surface contact, and due to the surface contact, a force is applied to the collet 814 toward the center 810A of the collet structure 810 to move the collet 814, so that the guide wire 1 or the catheter 2 penetrating the center 810A of the collet structure 810 can be stably pinched by the collet 814.

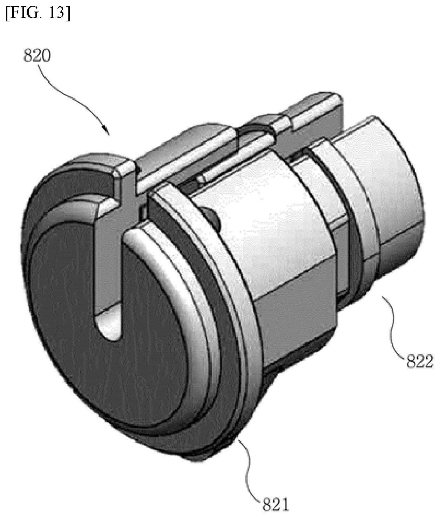

FIG. 13 is a perspective view of a plate spring structure in a module according to one embodiment of the present invention.

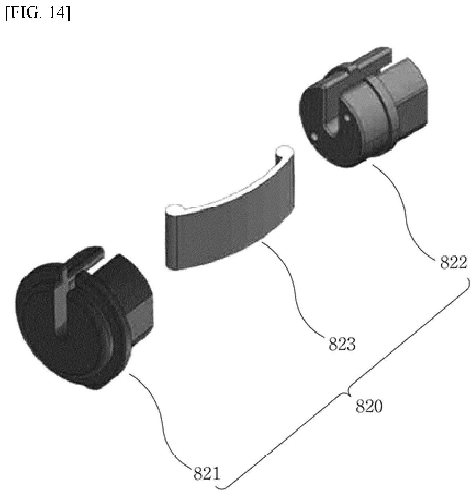

FIG. 14 is a perspective view of a plate spring structure before being assembled in a module according to one embodiment of the present invention.

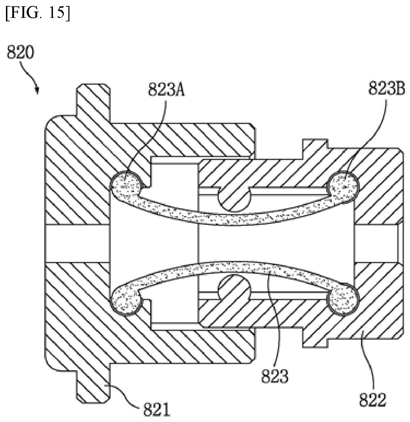

FIG. 15 is a cross-sectional view of a spring structure in a state of release from pinching in a module according to a modified embodiment of the present invention

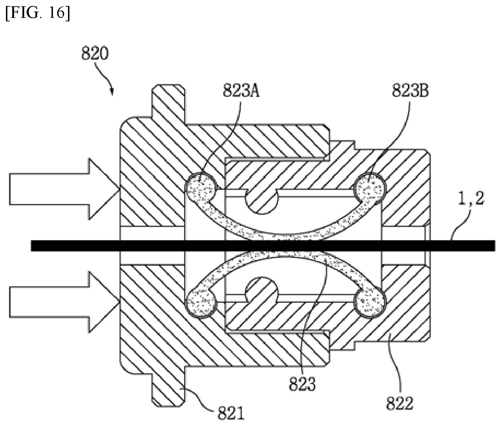

FIG. 16 is a cross-sectional view of a plate spring structure in a state of pinching in a module according to one embodiment of the present invention.

Referring to FIGS. 13 to 16, the plate spring structure 820 comprises a moving portion 821, a supporting portion 822, and one or more plate springs 823.

In the plate spring structure 820, the side on which the moving portion 821 is positioned is referred to as the front, and the side on which the supporting portion 822 is positioned is referred to as the rear.

Referring to FIG. 15, the plate spring 823 is disposed between the moving portion 821 and the supporting portion 822, and a first end 823A of the plate spring 823 is fixed to an inner surface of the moving portion 821, and a second end 823B of the plate spring 823 is fixed to an inner surface of the supporting portion 822.

Since the moving portion 821 is coupled to a straight extended portion of the rack 400, when the rack 400 is moved rearward, the moving portion 821 is also moved rearward, and when the rack 400 is moved forward, the moving portion 821 is also moved forward.

The support portion 822 is mechanically coupled to the second bevel gear 700, or the support portion 822 is formed as a feature integral with the second bevel gear 700. Due to the structure, the support portion 822 is rotated together with the rotation of the second bevel gear 700. The plate spring 823 is rotated together with the rotation of the second portion 812.

The one or more plate springs 823 are preferably two plate springs 823. FIGS. 15 and 16 illustrate two plate springs 823 disposed between the moving portion 821 and the supporting portion 822.

Referring to FIG. 16, when the rack 400 is moved rearward, the moving portion 821 is moved rearward in conjunction with the movement of the rack 400.

When the moving portion 821 is moved rearward, the first end 823A of the plate spring 823 fixed to the inner surface of the moving portion 821 is also moved rearward, and accordingly, the two plate springs 823 facing each other with the guide wire 1 or the catheter 2 interposed therebetween are subjected to bending deformation in a direction toward each other. Due to the bending deformation, the gap between the two plate springs 823 narrows, thereby pinching the guide wire 1 or the catheter 2 penetrating the center 820A of the plate spring structure 820.

In addition, as the distance by which the rack 400 is moved rearward increases, the distance by which the first end 823A of the plate spring 823 fixed to the inner surface of the moving portion 821 is moved rearward also increases, so that the degree of the bending deformation of the plate spring 823 increases, and the guide wire 1 or the catheter 2 is pinched more strongly.

The use of the plate spring structure 820 in this manner has the effect that the degree of the bending deformation of the plate spring 823 can be controlled, thereby allowing guide wires 1 or catheters 2 of various sizes to be pinched.

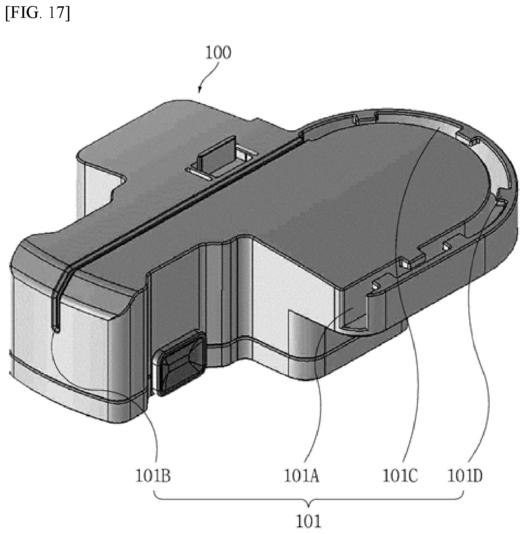

FIG. 17 is a perspective view of a guide wire drive cassette in a module according to one embodiment of the present invention.

In a guide wire drive cassette 100 in a module according to one embodiment of the present invention, a supply line 101 through which the guide wire 1 may be supplied is formed.

The supply line 101 comprises an inlet portion 101A where the guide wire 1 enters, an outlet portion 101B where the guide wire 1 exits, a curved portion 101C between the inlet portion 101A and the outlet portion 101B, and a protruding portion 101D.

The width of the supply line 101 is greater than the diameter of the guide wire 1, and the guide wire 1 is introduced through the inlet portion 101A, passes through the curved portion 101C, and exits through the outlet portion 101B.

In the supply line 101, one or more protruding portions 101D for preventing the guide wire 1 from easily departing from the supply line 101 are formed in the inlet portion 101A and the curved portion 101C.

Due to the curved portion 101C and the protruding portion 101D formed in the supply line 101, the guide wire drive cassette 100 has the effect of effectively preventing the guide wire 1 positioned in the supply line 101 from departing from the guide wire drive cassette 100.

### [DESCRIPTION OF REFERENCE NUMERALS]

1: guide wire
2: catheter
100: guide wire drive cassette
101: guide wire supply line
101A: inlet portion of guide wire supply line
101B: outlet portion of guide wire supply line
101C: curved portion of guide wire supply line
101D: protruding portion of guide wire supply line
200: catheter drive cassette
210: first catheter drive cassette
220: second catheter drive cassette
300: catheter fixing cassette
310: first catheter fixing cassette
320: second catheter fixing cassette
330: third catheter fixing cassette
400: rack
500: pinion
600: first bevel gear
700: second bevel gear
800: pinch module
810: collet structure
810A: center of collet structure
811: first portion of collet structure
811A: through hole at front end of first portion
812: second portion of collet structure
813: collet module
814: collet
814A, 814B: two or more contact points
814C: anti-slip groove
815: guide plate
815A: guide groove
816: biasing member
820: plate spring structure
820A: center of plate spring structure
821: moving portion
822: supporting portion
823: plate spring
823A: first end of plate spring
823B: second end of plate spring
900: housing
1000: module for guiding at least one of guide wire and catheter

## Claims

1. A module 1000 for guiding at least one of a guide wire 1 and a catheter 2,
the module 1000 comprising:
a housing 900 extending in a longitudinal direction from the front to the rear;
a guide wire drive cassette 100 for pinching the guide wire 1 to drive the guide wire 1;
at least one catheter drive cassette 200 for pinching the catheter 2 to drive the catheter 2; and,
at least one catheter fixing cassette 300 to which a proximal end of the catheter 2 is coupled,
wherein the guide wire drive cassette 100, the catheter drive cassette 200, and the catheter fixing cassette 300 are disposed in the housing 900,
the guide wire drive cassette 100 comprises a guide wire drive module for rotating the guide wire 1 in a state where the guide wire 1 is pinched,
the catheter drive cassette 200 comprises a catheter drive module for rotating the catheter 2 in a state where the catheter is pinched, and
the guide wire drive cassette 100 and the catheter drive cassette 200 can be moved along the longitudinal direction of the housing 900 by their respective actuators.

2. The module according to claim 1,
wherein the guide wire drive module and the catheter drive module each comprise a rack 400, a pinion 500, a first bevel gear 600, a second bevel gear 700, and a pinch module 800.

3. The module according to claim 2,
wherein the rack 400 is moved forward or rearward by rotation of the pinion 500, and
when the rack 400 is moved rearward, the guide wire 1 or the catheter 2 is pinched by the pinch module 800 in conjunction with the movement of the rack 400, and
when the rack 400 is moved forward, the guide wire 1 or the catheter 2 is released from being pinched by the pinch module 800 in conjunction with the movement of the rack 400.

4. The module according to claim 3,
wherein - in a state where the guide wire 1 is pinched by the pinch module 800 of the guide wire drive cassette 100,
the guide wire 1 is rotated by rotation of the pinch module 800 of the guide wire drive cassette 100, and
the guide wire 1 is moved forward or rearward by forward or rearward movement of the guide wire drive cassette 100 along the longitudinal direction of the housing 900, and
- in a state where the catheter 2 is pinched by the pinch module 800 of the catheter drive cassette 200,
the catheter 2 is rotated by rotation of the pinch module 800 of the catheter drive cassette 200, and
the catheter 2 is moved forward or rearward by forward or rearward movement of the catheter drive cassette 200 along the longitudinal direction of the housing 900.

5. The module according to claim 4,
wherein - only one of the forward or rearward movement of the guide wire 1 and the rotation of the guide wire 1 is performed, or both are performed simultaneously, and
- only one of the forward or rearward movement of the catheter 2 and the rotation of the catheter 2 is performed, or both are performed simultaneously.

6. The module according to claim 5,
wherein the guide wire drive cassette 100 is positioned at the rear of the catheter fixing cassette 300, and
the catheter fixing cassette 300 is positioned at the rear of the catheter drive cassette 200.

7. The module according to claim 6,
wherein, in a state where the catheter 2 is pinched by the pinch module 800 of the catheter drive cassette 200,
the catheter fixing cassette 300 is moved forward or rearward in conjunction with forward or rearward movement of the catheter drive cassette 200.

8. The module according to claim 7,
wherein, in a state where the catheter 2 is released from being pinched by the pinch module 800 of the catheter drive cassette 200,
the catheter drive cassette 200 is moved forward or rearward independently of the catheter fixing cassette 300.

9. The module according to any one of claims 1 to 8,
wherein the diameter of the guide wire 1 is smaller than the diameter of the catheter 2, the guide wire 1 penetrates the interior of the catheter 2, and
the driving of the guide wire 1 and the catheter 2 is performed independently of each other or in synchronization with each other.

10. The module according to any one of claims 2 to 8,
wherein the pinch module 800 is a collet structure 810 or a plate spring structure 820.

11. The module according to claim 10,
wherein the collet structure 810 comprises a first portion 811, a second portion 812, and a collet module 813,
the collet module 813 is disposed between the first portion 811 and the second portion 812, so as to face the inner side of the first portion 811 and be coupled to the second portion 812,
when the rack 400 is moved rearward, the first portion 811 is moved rearward in conjunction with the movement of the rack 400,
when the first portion 811 is moved rearward, the inner side of the first portion 811 and the outer side of one or more collets 814 of the collet module 813 come into contact with each other at one or more contact points, so that the collet 814 is moved in a direction toward the center 810A of the structure 810, and
the guide wire 1 or the catheter 2 penetrating the center 810A of the collet structure 810 is pinched by the collet 814.

12. The module according to claim 11,
wherein the inner side of the first portion 811 and the outer side of one or more collets 814 of the collet module 813 come into contact with each other at at least two contact points 814A, 814B, and
the at least two contact points 814A, 814B are spaced apart from each other along the longitudinal direction of the collet 814 on the outer side of the collet 814.

13. The module according to claim 11,
wherein at least one anti-slip groove 814C is formed along the longitudinal direction of the collet 814 on the inner side of the collet 814.

14. The module according to claim 11,
wherein a through hole 811A having a diameter smaller than the outer diameter of the collet 814 is formed at the front end of the first portion 811, and
when the first portion 811 is moved rearward, the through hole 811A of the first portion 811 and the outer side of the collet 814 come into contact with each other.

15. The module according to claim 10,
wherein the plate spring structure 820 comprises a moving portion 821, a supporting portion 822, and one or more plate springs 823,
the plate spring 823 is disposed between the moving portion 821 and the supporting portion 822, such that a first end 823A of the plate spring 823 is fixed to an inner surface of the moving portion 821, and a second end 823B of the plate spring 823 is fixed to an inner surface of the supporting portion 822,
when the rack 400 is moved rearward, the moving portion 821 is moved rearward in conjunction with the movement of the rack 400, and
when the moving portion 821 is moved rearward, the plate springs 823 are subjected to bending deformation in a direction toward each other, so that the guide wire 1 or the catheter 2 penetrating the center 810A of the plate spring structure 820 is pinched.

16. The module according to claim 15,
wherein the degree of the bending deformation of the plate spring 823 increases as the distance by which the rack 400 is moved rearward increases.

17. The module according to any one of claims 1 to 8,
wherein a guide wire supply line 101 through which the guide wire 1 may be supplied is formed in the guide wire drive cassette 100, and
the guide wire supply line 101 comprises an inlet portion 101A where the guide wire 1 enters, an outlet portion 101B where the guide wire 1 exits, a curved portion 101C between the inlet portion 101A and the outlet portion 101B, and a protruding portion 101D.

18. The module according to any one of claims 1 to 8,
wherein the distance by which the guide wire drive cassette 100 and the catheter drive cassette 200 can move forward or rearward along the longitudinal direction of the housing 900 is limited to a distance at which the guide wire 1 or the catheter 2 does not buckle.

19. The module according to any one of claims 1 to 8,
wherein the at least one catheter drive cassette 200 comprises a plurality of catheter drive cassettes, and
the at least one catheter fixing cassette 300 comprises a plurality of catheter fixing cassettes.

20. The module according to any one of claims 1 to 8,
wherein the at least one catheter drive cassette 200 comprises one first catheter drive cassette 210 and one second catheter drive cassette 220,
the at least one catheter fixing cassette 300 comprises one first catheter fixing cassette 310 and one second catheter fixing cassette 320,
the guide wire drive cassette 100 is disposed at the rear of the first catheter fixing cassette 310,
the first catheter fixing cassette 310 is disposed at the rear of the first catheter drive cassette 210,
the first catheter drive cassette 210 is disposed at the rear of the second catheter fixing cassette 320,
the second catheter fixing cassette 320 is disposed at the rear of the second catheter drive cassette 220,
a proximal end of the first catheter is coupled to the first catheter fixing cassette 310,
a proximal end of the second catheter is coupled to the second catheter fixing cassette 320,
the diameter of the guide wire 1 is smaller than the diameter of the first catheter, the guide wire 1 penetrates the interior of the first catheter,
the diameter of the first catheter is smaller than the diameter of the second catheter, and the first catheter penetrates the interior of the second catheter.

21. The module according to claim 20,
wherein the at least one catheter fixing cassette 300 comprises one third catheter fixing cassette 330,
the third catheter fixing cassette 330 is disposed at a front end of the housing 900,
the second catheter drive cassette 220 is disposed at the rear of the third catheter fixing cassette 330,
a proximal end of the third catheter is coupled to the third catheter fixing cassette,
the diameter of the second catheter is smaller than the diameter of the third catheter, and the second catheter penetrates the interior of the third catheter.
